# EUROPEAN PATENT APPLICATION

(11) **EP 4 413 992 A1**
(43) Date of publication of application: **14.08.2024**
(21) Application number: 22878885.7
(22) Date of filing: 05.10.2022
(51) Int. Cl.: A61K 38/10, A61P 35/00, A61P 35/04

(54) **COMPOSITION FOR ENHANCING ACTIVITY OF NATURAL KILLER CELLS**

(30) Priority: 05.10.2021 KR 20210131745
(71) Applicant: Ingenium Therapeutics, Daejeon 34054 (KR)
(72) Inventor: CHOI, In Pyo, Daejeon 34141 (KR); JUNG, Haiyoung, Daejeon 34141 (KR); KIM, Hanna, Daejeon 34141 (KR); YOON, Suk Ran, Daejeon 34141 (KR); BYUN, Jae Eun, Daejeon 34141 (KR)
(74) Representative: Moré, Solveig Helga
(86) International application number: PCT/KR2022/014996
(87) International publication number: WO 2023/059064

(57) **Abstract**

The present invention relates to a composition for enhancing the activity of natural killer cells, and provides a composition for enhancing the activity of natural killer cells, the composition containing, as an active ingredient, a peptide having an amino acid sequence of SEQ ID NO: 1. The peptide having the amino acid sequence of SEQ ID NO: 1 according to the present invention not only increases the cytotoxicity of natural killer cells but also reduces the fratricide increased by TGF-β, and further improves the sensitivity of the natural killer cells to cancer cells. Thus, the peptide having the amino acid sequence of SEQ ID NO: 1 can be effectively used as an active ingredient for an anticancer drug or an anticancer adjuvant.

## Description

### [Technical Field]

The present invention relates to a composition for enhancing the activity of natural killer cells, and more specifically, to a composition for recovering the activity of natural killer cells decreased by TGF-β.

### [Background Art]

Natural killer cells (NK cells) are lymphocytes present in the bone marrow, lymph nodes, and peripheral blood, and account for approximately 10 to 15% of all lymphocytes. Phenotypes of NK cells are characterized to be CD3 negative and CD56 positive, and NK cells, as immune cells with the ability to kill cancer cells or virus-infected cells, play an important role in the innate immune responses. The functions of the NK cells are regulated by the interaction between cell surface receptors and corresponding target cell ligands without stimulation of specific antigens, and these receptors are largely divided into an activating receptor and an inhibitory receptor. Representative activating receptors that regulate the functions of the NK cells by transmitting activation signals to the NK cells include natural cytotoxicity receptors (NCRs) such as NKp30, NKp44, NKp46, etc., NKG2D, etc. The activated NK cells may destroy target cells by synthesizing various granules and secreting the granules outside the cells. Among the representative proteins contained in the granules, perforin and granzyme play a major role in destroying target cells. The perforin gathers on the cell membrane of the target cell to form a complex and punctures the cell membrane to cause cell lysis, and the granzyme is introduced into the cells through the holes generated in the cells to not only activate caspase, but also cause cell death through various mechanisms. In addition, the NK cells induce apoptosis of cancer cells by binding to death receptors of cancer cells using death ligands such as FasL and TRAIL. The binding of these receptors activates caspase-8 and caspase-10 in cancer cells to activate caspase-3 and caspase-7, thereby causing apoptosis.

Due to these characteristics, the NK cells have been applied to anticancer therapy. However, most successful treatments were limited to blood cancer, and the effect thereof was minimal in solid cancer. In the treatment of solid cancer, it is difficult for the NK cells to migrate and infiltrate the solid cancer tissue. Furthermore, even if the NK cells are introduced to cancer tissue, the NK cells do not exhibit an appropriate anticancer effect due to an immunosuppressive microenvironment of the cancer tissue.

Meanwhile, TGF-β is a cytokine with immunosuppressive activity and suppresses the anticancer activity of T cells or NK cells. In particular, in the solid cancer, TGF-β is produced and secreted to suppress the immune functions within the microenvironment of cancer, thereby helping the growth of cancer tissue. TGF-β secretion in metastatic cancer is closely related to the poor prognosis of cancer. Actually, the anticancer activity of the NK cells is enhanced by inhibiting TGF-β production or signaling. TGF-β inhibits the production of IFN-γ by NK cells and suppresses the killing ability by CD16.

Accordingly, there is a need for research and development of ingredients to restore the activity of NK cells suppressed by TGF-β or to enhance the anticancer immune activity against solid cancer or metastatic cancer.

### [Disclosure]

### [Technical Problem]

An object of the present invention is to provide a composition for enhancing the activity of NK cells.

### [Technical Solution]

An aspect of the present invention provides a composition for enhancing the activity of NK cells including a peptide having an amino acid sequence of SEQ ID NO: 1 or a polynucleotide encoding the peptide as an active ingredient.

Another aspect of the present invention provides a pharmaceutical composition for preventing or treating cancer including a peptide having an amino acid sequence of SEQ ID NO: 1 or a polynucleotide encoding the peptide as an active ingredient.

Yet another aspect of the present invention provides a cellular therapeutic agent for preventing or treating cancer including NK cells treated with a peptide having an amino acid sequence of SEQ ID NO: 1 or a polynucleotide encoding the peptide.

Yet another aspect of the present invention provides a method for enhancing the activity of NK cells including treating NK cells with a peptide having an amino acid sequence of SEQ ID NO: 1 or a polynucleotide encoding the peptide.

Yet another aspect of the present invention provides a method for treating cancer including administering a pharmaceutical composition for preventing or treating cancer including a peptide having an amino acid sequence of SEQ ID NO: 1 or a polynucleotide encoding the peptide to a subject in need of treatment.

Yet another aspect of the present invention provides a method for treating cancer including (a) treating NK cells with a peptide having an amino acid sequence of SEQ ID NO: 1 or a polynucleotide encoding the peptide; and (b) administering the NK cells of step (a) to a subject.

Yet another aspect of the present invention provides a method for treating cancer including administering, to a subject, a pharmaceutical composition for preventing or treating cancer including a peptide having an amino acid sequence of SEQ ID NO: 1 or a polynucleotide encoding the peptide as an active ingredient; and NK cells.

### [Advantageous Effects]

According to the present invention, the peptide having the amino acid sequence of SEQ ID NO: 1 not only increases the cytotoxicity of NK cells, but also reduces the fratricide increased by TGF-β, and further improves the sensitivity of the NK cells to cancer cells. Thus, the peptide of the present invention can be effectively used as an active ingredient for an anticancer drug or an anticancer adjuvant.

### [Description of Drawings]

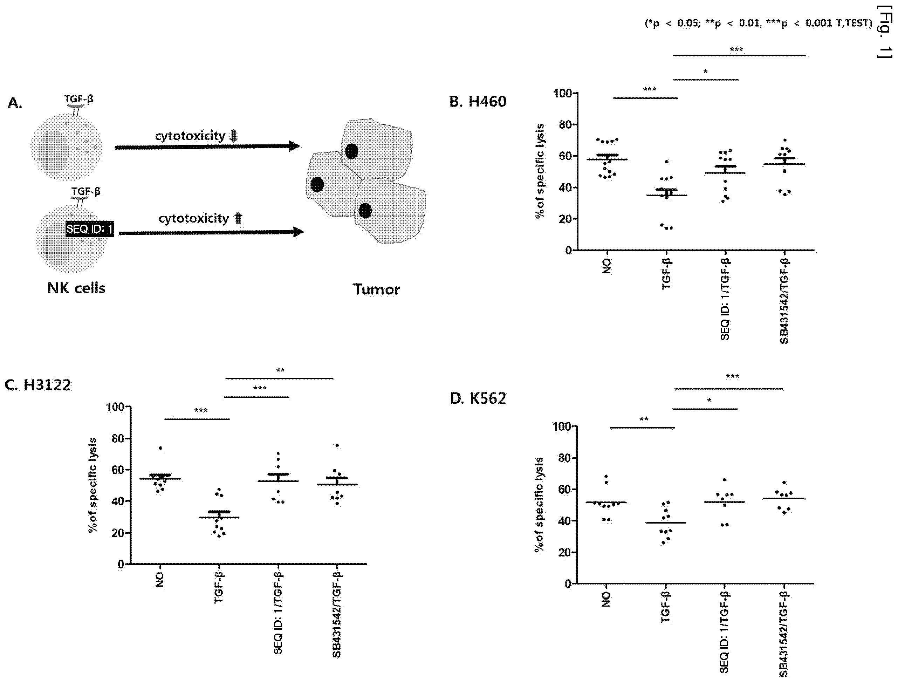

FIG. 1 illustrates results of confirming that the cytotoxicity ofNK92 cells reduced by TGF-β is recovered by treatment with a peptide consisting of an amino acid sequence of SEQ ID NO: 1. FIG. 1A is a diagram schematically describing a principle of an effect of the peptide on NK cells, and FIGS. 1B, 1C, and 1D illustrate experimental results of measuring the cytotoxicity of NK92 cells to a lung cancer cell line H460, a lung cancer cell line H3122, and a blood cancer cell line K562, respectively.

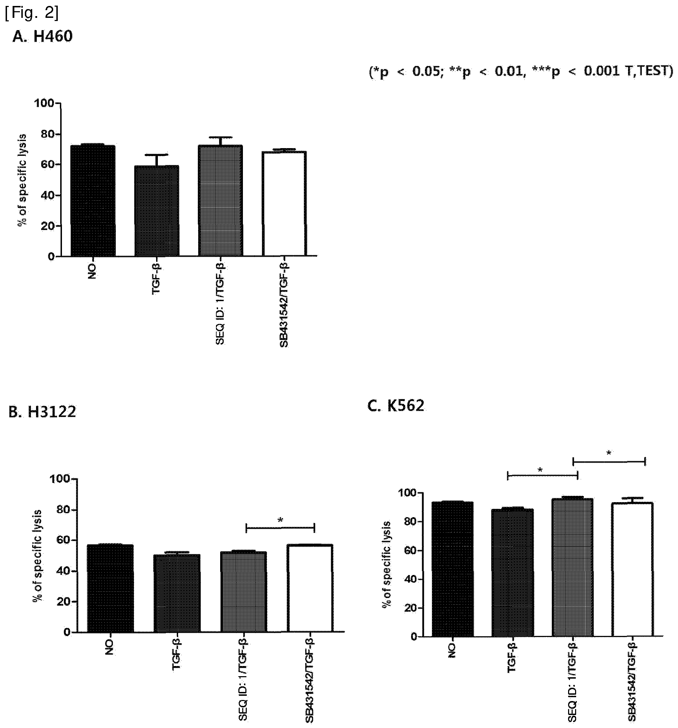

FIG. 2 illustrates results of confirming that the cytotoxicity of primary NK cells reduced by TGF-β is recovered by treatment with a peptide consisting of an amino acid sequence of SEQ ID NO: 1. FIGS. 2A, 2B, and 2C illustrate experimental results of measuring the cytotoxicity of primary NK cells to a lung cancer cell line H460, a lung cancer cell line H3122, and a blood cancer cell line K562, respectively.

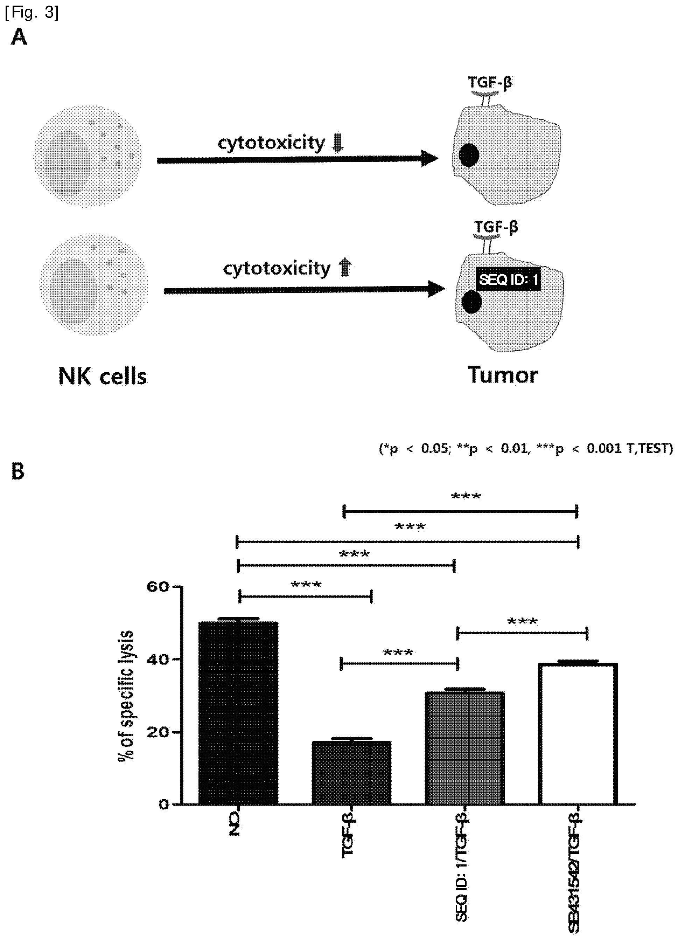

FIG. 3 is a diagram illustrating the recovery of the reduced killing ability of NK92 to a lung cancer cell line H460 treated with TGF-β and sensitivity decreased by the peptide having the amino acid of SEQ ID NO: 1. FIG. 3A is an explanatory diagram schematically illustrating an NK92 cytotoxicity test after treating H460 cells, a lung cancer cell line, with TGF-β, and FIG. 3B illustrates that when H460 cells are treated with TGF-β, the killing ability of NK92 is reduced by less than half, and the reduced killing ability is recovered by the peptide.

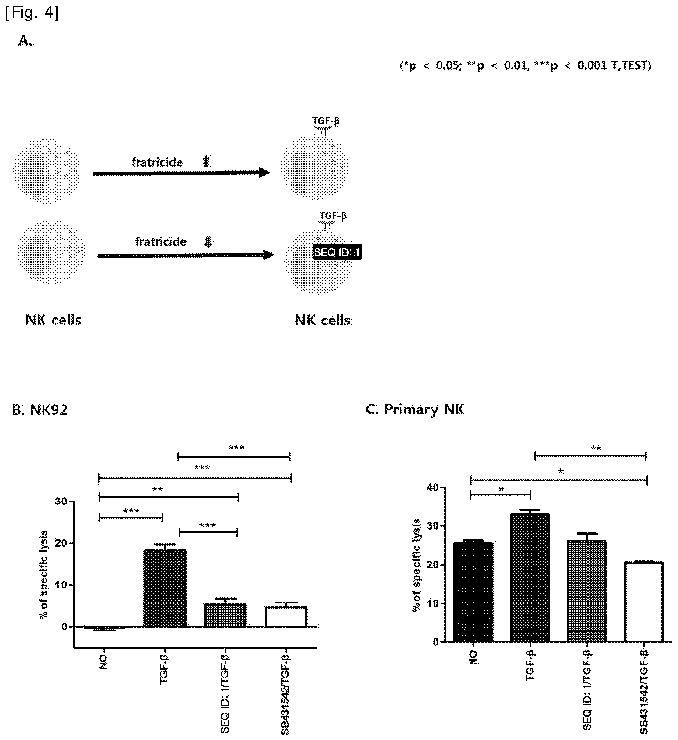

FIG. 4 illustrates results of confirming an increase in fratricide between NK cells by TGF-β and a decrease in fratricide by the peptide having the amino acid of SEQ ID NO: 1. FIG. 4A schematically illustrates an effect of reducing fratricide of NK cells using NK92 reacted with added TGF-β as target cells, and FIGS. 4B and 4C illustrate an increase in fratricide of NK-92 cells in which NK92 and primary NK cells react with TGF-β and a decrease in fratricide by the peptide, respectively.

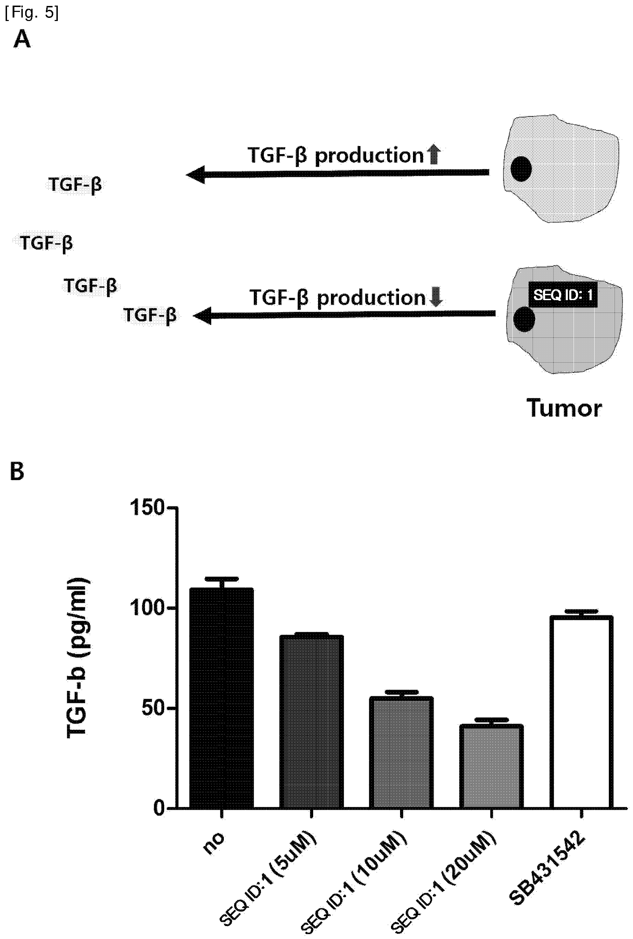

FIG. 5 is a diagram illustrating that the TGF-β expression in lung cancer cells is inhibited by the peptide having the amino acid of SEQ ID NO: 1. FIG. 5A illustrates an effect of the peptide on decreasing the TGF-β expression in lung cancer cell line H460 cells, and FIG. 5B illustrates reducing the expression of TGF-β in H460 cells by the peptide.

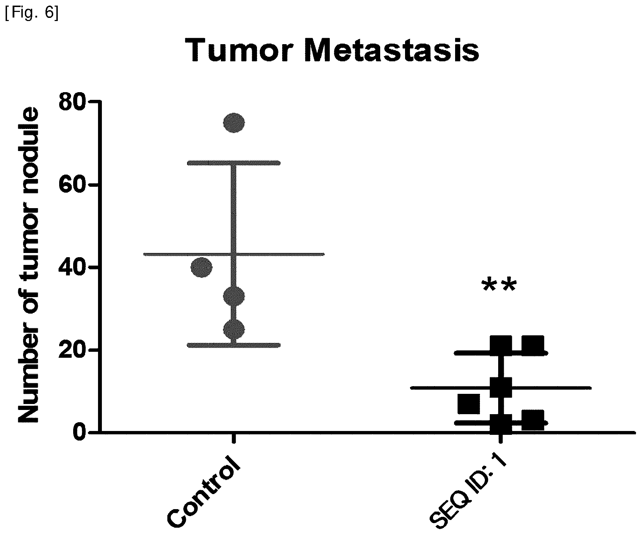

FIG. 6 illustrates that the peptide inhibits lung metastasis of B16F10 melanoma in an animal model.

### [Modes of the Invention]

Hereinafter, the present invention will be described in detail.

An aspect of the present invention provides a composition for enhancing the activity of natural killer cells (NK cells).

The composition for enhancing the activity of the NK cells of the present invention includes as an active ingredient a peptide having an amino acid sequence of SEQ ID NO: 1 or a functional equivalent thereof, or a polynucleotide encoding the peptide.

The peptide may include the amino acid sequence of SEQ ID NO: 1 or functional equivalent thereof, or may consist of only the amino acid sequence of SEQ ID NO: 1 or a functional equivalent thereof. The functional equivalent of the peptide may have the activity of the peptide having the amino acid sequence of SEQ ID NO: 1, in which some or all of 13 amino acids constituting the amino acid sequence of SEQ ID NO: 1 are substituted with other amino acids, some of the 13 amino acids are deleted, at least one amino acid is added to the 13 amino acids, or some or all of the 13 amino acids are modified. The substitution of the amino acids may be a conservative substitution, and for example, mutual substitution between aliphatic uncharged amino acids (Gly, Ala, Val, Leu, Ile), mutual substitution between hydrophilic uncharged amino acids (Ser, Thr, Asn, Gln), mutual substitution between aromatic amino acids (Phe, Tyr, Trp), mutual substitution between acidic amino acids (Asp, Glu), mutual substitution between basic amino acids (His, Lys, Arg), mutual substitution between non-polar uncharged amino acids (Cys, Met, Pro), etc. The deletion or addition of the amino acids may occur, for example, in a portion that is not directly involved in a portion that does not affect the activity of the peptide having the amino acid sequence of SEQ ID NO: 1. Particularly, the addition of the amino acids may be the insertion of at least one amino acid in the middle of the amino acid sequence of SEQ ID NO: 1, as well as the N-terminus or C-terminus of the amino acid sequence of SEQ ID NO: 1. In addition, the modification of the amino acids may be phosphorylation, acetylation, methylation, glycosylation, etc. of some or all of the 13 amino acids constituting the amino acid sequence of SEQ ID NO: 1. In some cases, the functional equivalent may have at least 60% homology, at least 65%, at least 70%, at least 75%, at least 80% or at least 85%, for example, at least 90% of homology with the amino acid sequence of SEQ ID NO: 1.

The polynucleotide may encode the peptide, and may, for example, have a base sequence of SEQ ID NO: 2 or a base sequence equivalent thereto. The equivalent base sequence is different from a base sequence of SEQ ID NO: 2, but means that the sequence of the encoding peptide is the same as the base sequence of SEQ ID NO: 2. In some cases, the equivalent base sequence may have at least 60%, at least 70%, at least 80%, or at least 90%, for example, at least 95% of homology with the base sequence of SEQ ID NO: 2.

The polynucleotide may be included in a vector. The vector is preferably capable of self-replication, and further, may express more preferably the polynucleotide, and may be, for example, a plasmid, cosmid, phage, etc.

In addition, the vector including the polynucleotide may be transformed into a host cell. The host cell may preferably express a peptide having the amino acid sequence of SEQ ID NO: 1 or its equivalent from the polynucleotide contained in the vector, and further, may more preferably secrete the peptide expressed as above to the outside of the cell.

The NK cells are cytotoxic lymphocytes that constitute a major component of the innate immune system, and are defined as large granular lymphocytes (LGLs) and play an important role in the innate and adaptive immune systems. The NK cells may include not only mature NK cells but also natural killer progenitor cells. In addition, the NK cells may be derived from mammals, and the mammals may be humans, monkeys, goats, sheep, rats, mice, etc., and especially humans, monkeys, or mice.

The activity of the NK cells may include the ability to kill target cells such as cancer cells or virus-infected cells, the degranulation of the NK cells, or stimulation of activating receptors or inactivation of inhibitory receptors of the NK cells. At this time, the activating receptors may be NKG2D, 2B4, DNAM-1, NCRs, etc., and the inhibitory receptors may be PD-1, LAG-3, TIM-3, etc.

The improving of the activity of the NK cells means an increase in cytotoxicity of the NK cells to target cells, a decrease in fratricide, improvement in sensitivity to target cells, etc. The increase in cytotoxicity of the NK cells may be increased expression or secretion of substances such as perforin, granzyme, and interferon, which are involved in cell death of target cells, or increased degranulation of granules containing the substances. In particular, the perforin may be perforin-1, perforin-2, etc., the granzyme may be granzyme A, granzyme B, granzyme H, granzyme K, granzyme M, etc., and the interferon may be type 1 interferon such as interferon-α, interferon-β, interferon-κ, and interferon-ω, type 2 interferon such as interferon-γ, or type 3 interferon such as interferon-L1. In addition, the fratricide refers to the cytotoxicity between the NK cells, and in other words, means a phenomenon in which the NK cells themselves serve as an effector cell and a target cell at the same time. The fratricide between the NK cells may be increased by TGF-β, and a decrease in fratricide of the NK cells means a decrease in the degree to which the NK cells recognize other NK cells as target cells. In addition, the improving of the sensitivity of the NK cells to target cells means that the NK cells may more easily recognize target cells.

In specific embodiments of the present invention, by treatment with the peptide consisting of the amino acid sequence of SEQ ID NO: 1, it is confirmed that the cytotoxicity of NK92 or primary NK cells decreased by TGF-β to a lung cancer cell line and a blood cancer cell line is recovered (see FIGS.1 and 2), the sensitivity of NK92 or primary NK cells decreased by TGF-β to lung cancer cell lines is recovered (see FIG. 3), but the fratricide between the NK cells increased by TGF-β is decreased (see FIG. 4). Accordingly, the peptide having the amino acid sequence of SEQ ID NO: 1 or the functional equivalent thereof of the present invention, or the polynucleotide encoding the peptide of the present invention may be used as an active ingredient to enhance the activity of NK cells.

Therefore, another aspect of the present invention provides a method for enhancing the activity of NK cells including treating NK cells with the peptide having the amino acid sequence of SEQ ID NO: 1 of the present invention or the polynucleotide encoding the peptide of the present invention.

Meanwhile, the composition for enhancing the activity of NK cells may be used as an anticancer drug or anticancer adjuvant. Therefore, yet another aspect of the present invention provides a pharmaceutical composition for preventing or treating cancer, in which the pharmaceutical composition for preventing or treating cancer may include a peptide having an amino acid sequence of SEQ ID NO: 1 or a functional equivalent thereof, or a polynucleotide encoding the peptide as an active ingredient.

In a specific embodiment of the present invention, it is confirmed that when the peptide consisting of the amino acid sequence of SEQ ID NO: 1 is administered to an animal model transplanted with melanoma cells, the number of nodules of cancer metastasized to the lungs is reduced compared to a negative control group (see FIG. 6). Accordingly, the peptide having the amino acid sequence of SEQ ID NO: 1 or the functional equivalent thereof of the present invention, or the polynucleotide encoding the peptide of the present invention may be used as an active ingredient for preventing or treating cancer.

The cancer generally refers to a physiological condition in mammals characterized by uncontrolled cell growth, and refers to a condition in which a problem occurs in the regulation function of normal division, differentiation, and death of cells, and thus the cells are abnormally hyperproliferated and infiltrated to surrounding tissues and organs to form lumps and destroy or deform existing structures. The cancer may be solid cancer or metastatic cancer, and for example, colorectal cancer including colon cancer and rectal cancer, breast cancer, uterine cancer, cervical cancer, ovarian cancer, prostate cancer, brain tumor, head and neck carcinoma, melanoma, myeloma, leukemia, lymphoma, stomach cancer, lung cancer, pancreatic cancer, liver cancer, esophageal cancer, small intestine cancer, perianal cancer, fallopian tube carcinoma, endometrial carcinoma, vaginal carcinoma, vulvar carcinoma, Hodgkin's disease, bladder cancer, kidney cancer, ureteral cancer, renal cell carcinoma, renal pelvic carcinoma, bone cancer, skin cancer, head cancer, cervical cancer, skin melanoma, intraocular melanoma, endocrine cancer, thyroid cancer, parathyroid cancer, adrenal cancer, soft tissue sarcoma, urethral cancer, penile cancer, central nervous system (CNS) tumor, primary CNS lymphoma, spinal cord tumor, brainstem glioma, pituitary adenoma, etc. Particularly, for the types of cancer, TGF-β may be overexpressed or overactivated.

The prevention refers to all actions that inhibit or delay the occurrence or progression of cancer, and the treatment refers to all effects that not only kill cancer cells, but also prevent cancer cells from getting worse, such as reduction in the growth of cancer cells, reduction in recurrence, reduction in formation of immune system anticancer activity and immune memory against cancer cells, or reduction in invasion of cancer cells, but are not limited thereto.

The composition may further include other active ingredients that exhibit the anticancer activity, in addition to the peptide having the amino acid sequence of SEQ ID NO: 1 or the functional equivalent thereof, or the polynucleotide encoding the peptide.

In addition, the composition may further include a pharmaceutically acceptable carrier or additive, in addition to the active ingredients.

The meaning of 'pharmaceutically acceptable' means that the composition does not inhibit the activity of the active ingredient and does not have more than adaptable toxicity of an application (prescription) target. The carrier is defined as a compound that facilitates the addition of the compound into cells or tissue.

The peptide having the amino acid sequence of SEQ ID NO: 1 or the functional equivalent thereof of the present invention, or the polynucleotide encoding the peptide of the present invention may be administered alone or in combination with any convenient carrier, etc., and such dosage form may be administered as a single administration or repeated administration. The composition may be solid formulations or liquid formulations. The solid formulations include powders, granules, tablets, capsules, suppositories, and the like, but are not limited thereto. The solid formulations may include a carrier, a flavoring agent, a binder, a preservative, a disintegrant, a lubricant, a filler, etc., but are not limited thereto. The liquid formulations include solutions such as water and a propylene glycol solution, suspensions, emulsions, and the like, but are not limited thereto and may be prepared by adding suitable coloring agents, flavoring agents, stabilizers, viscous agents, etc. For example, the powders may be prepared by simply mixing the peptide having the amino acid sequence of SEQ ID NO: 1 or the functional equivalent thereof or the polynucleotide encoding the peptide, as the active ingredient of the present invention, with a suitable pharmaceutically acceptable carrier such as lactose, starch, and microcrystalline cellulose. The granules may be prepared by mixing the peptide having the amino acid sequence of SEQ ID NO: 1 or the functional equivalent thereof or the polynucleotide encoding the peptide, a suitable pharmaceutically acceptable carrier, and a suitable pharmaceutically acceptable binder such as polyvinylpyrrolidone and hydroxypropylcellulose, and then using a wet granulation method using a solvent such as water, ethanol, and isopropanol, or a dry granulation method using compression force. Further, the tablets may be prepared by mixing the granules with a suitable pharmaceutically acceptable lubricant such as magnesium stearate, and then tableting the mixture using a tablet machine.

The peptide having the amino acid sequence of SEQ ID NO: 1 or the functional equivalent thereof, or the polynucleotide encoding the peptide of the present invention may be administered with an oral agent, an injection (e.g., intramuscular injection, intraperitoneal injection, intravenous injection, infusion, subcutaneous injection, implant), an inhalant, a nasal injection, a vaginal agent, a rectal agent, a sublingual agent, a transdermal agent, a topical agent, or the like according to a disease to be treated and a condition of a subject, but is not limited thereto. Depending on a route of administration, the active ingredient may be prepared into a suitable dosage unit formulation including pharmaceutically acceptable carrier, additive, and vehicle, which are commonly used and non-toxic.

The composition may be administered daily in an amount of about 0.0001 mg/kg to about 10 g/kg, and administered in a daily dose of about 0.001 mg/kg to about 1 g/kg. However, the dose may vary depending on the degree of purification of the mixture, the patient's condition (age, sex, body weight, etc.), the severity of the condition to be treated, and the like. If necessary, for convenience, the total daily dose may be divided and administered several times during a day.

Yet another aspect of the present invention provides a cellular therapeutic agent for preventing or treating cancer including NK cells treated with a peptide having an amino acid sequence of SEQ ID NO: 1 or a polynucleotide encoding the peptide.

The term "cellular therapeutic agent" refers to a medicine (US FDA regulations) used for the purposes of treatment, diagnosis, and prevention with cells and tissues prepared through isolation, culture, and special manipulation from a subject, and means a medicine used for treatment, diagnosis and prevention through a series of actions, such as *ex vivo* proliferating and selecting living autologous, allogeneic, or xenogenic cells to restore the function of cells or tissues, changing the biological characteristics of cells by other methods, and the like.

In the present invention, the cellular therapeutic agent refers to a therapeutic agent used for the treatment of cancer, and may be used as a cellular therapeutic agent for the treatment and prevention of cancer by including as an active ingredient NK cells with increased killing ability to cancer cells by treating the peptide having the amino acid sequence of SEQ ID NO: 1 or the polynucleotide encoding the peptide.

Yet another aspect of the present invention provides a method for treating cancer including administering a pharmaceutical composition for preventing or treating cancer including the peptide having the amino acid sequence of SEQ ID NO: 1 or the polynucleotide encoding the peptide as the active ingredient to a subject.

Yet another aspect of the present invention provides a method for treating cancer including the following steps:
(a) treating NK cells with the peptide consisting of the amino acid sequence of SEQ ID NO: 1 of the present invention or the polynucleotide encoding the peptide of the present invention; and
(b) administering the NK cells of step (a) to a subject.

Since the method of the present invention uses the NK cells with increased killing ability to cancer cells by treating the peptide of the present invention or the polynucleotide encoding the peptide of the present invention, duplicate contents will be omitted in order to avoid excessive complexity of the specification.

Hereinafter, the present invention will be described in detail by Examples.

However, the following Examples specifically illustrate the present invention, and the contents of the present invention are not limited by the following Examples.

### [Example 1]

### [1-1] NK92 cell culture

NK92 cells were cultured in a 37°C CO₂ incubator (water jacketed incubator, Thermo electron corporation) supplied with 5% CO₂ by adding an α-MEM (Welgene) culture medium added with 12.5% fetal bovine serum (FBS, Corning, united states origin, 35-015-CV) and 12.5% horse serum (Gibco) according to ATCC culture conditions with 0.2 mM myo-inositol, 0.1 mM 2-mercaptoethanol, 0.02 mM folic acid, 1% antibiotics-antimycotic (Gibco) and IL-2 cytokine (Peprotech) at a concentration of 20 ng/ml to be used as a culture medium.

The cells were cultured in about 20 to 30 ml in a T-75 flask (Thermo) at a concentration of 1 × 10⁵ cells/ml, and centrifuged at 1000 rpm for 5 minutes every 2 to 3 days using a centrifuge (Beckman Coulter) to remove the supernatant, and then the culture medium was replaced.

### [1-2] Primary NK cell culture

2 µl of Rosettesep Human CD3 depletion cocktail (STEMCELL technologies, catalog #15621) per 1 ml of blood was added to umbilical cord blood (IRB approval number P01-201610-31-002) donated from the hospital and reacted for 20 minutes. The blood reacted with Ficoll (GE healthcare, Ficoll-paque premium) dispensed at 15 ml each was slowly increased by 35 ml. At this time, it could be noted that the blood and the Ficoll layer were not mixed. To separate the cell layer from the blood, red blood cells and CD3 cells were isolated and discharged from the blood by centrifugation under conditions of2000 rpm and Decel 0 for 25 minutes using a centrifuge (Beckman Coulter). The CD3-isolated cells reacted with 10 ml of an ACK buffer for 10 minutes to remove remaining red blood cells, and then washed twice using phosphate-buffered saline (PBS) added with 2% FBS. The washing was performed by centrifuging at 1500 rpm for 5 minutes using a centrifuge (Beckman Coulter) to remove the supernatant. The cells obtained above were used to be cultured as NK cells.

The NK cells were cultured by adding alpha-MEM (Welgene) added with 10% FBS (Corning, United States origin, 35-015-CV) with 10 ng/ml IL-15 (Peprotech), 10 ng/ml IL-21 (Peprotech), 10⁶ M hydrocortisone (Peprotech) and 1% antibiotics-antimycotic (Gibco). The cells were cultured in a 6-well plate at a cell culture concentration of 2 × 10⁶ cells/ml, and centrifuged every 2 to 3 days at 1200 rpm for 5 minutes using a centrifuge (Beckman Coulter) to remove the supernatant, and then the culture medium was replaced.

The degree of differentiation of the NK cells was confirmed every 3 days using a FACSCanto II (BD Biosciences) flow cytometer. For the FACS flow cytometry, 2 × 10⁴ cells were dispensed into a FACS tube (Falcon), centrifuged at 1500 rpm for 3 minutes with PBS added with FBS (FACS buffer) using a centrifuge (Beckman Coulter) and washed to remove the supernatant, and then added with 100 µl of the FACS buffer again. 1 µl each of CD56 APC (BD) antibody and CD3 PE (BD) antibody were added, stained at 4°C for 20 minutes, and then added with 500 µl each of the FACS buffer, and centrifuged at 1500 rpm for 3 minutes using a centrifuge (Beckman Coulter) and washed. After removing the supernatant, 200 µl of the FACS buffer was added and the analysis was performed with FACSCanto II (BD Biosciences).

CD56 differentiation was confirmed to be about 90% after 7 to 9 days of culture, and an experiment was performed using primary NK cells differentiation-confirmed.

### [1-3] Cell culture of lung cancer cell lines H460 and H3122

In a cytotoxicity test, lung cancer cell lines H460 and H3122 were cultured and used as target cell lines. The H460 and H3122 cells were cultured in a 37°C CO₂ incubator (water jacketed CO₂ incubator, Thermo electron corporation) supplied with 5% CO₂ using a culture medium added with an RPMI (Welgene) culture medium added with 10% FBS (Corning, united states origin, 35-015-CV) and 1% antibiotics-antimycotic (Gibco) according to ATCC culture conditions. About 10 ml of the cells were cultured at a concentration of 2 × 10⁵ cells/ml in a 100 mm × 20 mm cell culture dish (Corning). The cells were observed and the culture medium was replaced every 2 to 3 days, and when the culture medium of H460 and H3122 was replaced, the supernatant was fully removed and reacted with 2 ml of 0.025% Trysin-EDTA (Gibco) for 3 minutes at 37°C, and then adherent cells were detached and centrifuged at 1000 rpm for 5 minutes using a centrifuge (Beckman Coulter), and a new culture medium was replaced.

### [1-4] Cell culture of blood cancer cell line K562

In a cytotoxicity test, a blood cancer cell line K562 was cultured and used as a target cell line. The K562 cells were cultured in a 37°C CO₂ incubator (water jacketed CO₂ incubator, Thermo electron corporation) supplied with 5% CO₂ using a culture medium added with an RPMI (Welgene) culture medium added with 10% FBS (fetal bovine serum, Corning, United States origin, 35-015-CV) and 1% antibiotics-antimycotic (Gibco) according to ATCC culture conditions. About 20 ml of the cells were cultured at a concentration of 2 × 10⁵ cells/ml in a 75T flask (Corning). The culture medium was replaced every 2 to 3 days.

### [1-5] Preparation of peptide having amino acid sequence of SEQ ID NO: 1

In addition, a peptide consisting of 13 amino acids of SEQ ID NO: 1 was synthesized by Peptron (Daejeon, Korea) and used in the following experiments.

### [Example 2]

### Recovery of cytotoxicity of NK cells decreased by TGF

How the peptide prepared in Examples [1-5] affected the cytotoxicity of NK cells decreased by TGF-β was confirmed.

### [2-1] Preparation of effector cells

To this end, first, 1 × 10⁵ cells/ml of NK92 cells or primary NK cells were added with 20 µM the peptide of Example [1-5] or 5 µM a TGF-β signal inhibitor SB431542, respectively, cultured at 37°C for 2 hours, and then added with 10 ng/ml of TGF-β, and cultured again for 24 hours under the same conditions, respectively. Then, the cells were added with 20 µM the peptide of Example [1-5] or 5 µM SB431542, cultured at 37°C for 2 hours, and then added with 10 ng/ml of TGF-β, and cultured under the same conditions for 24 hours, respectively. The NK92 cells and primary NK cells cultured above were used as effector cells in the cytotoxicity test.

### [2-2] Preparation of target cells

Meanwhile, lung cancer cell lines H460 and H3122 and a blood cancer cell line K562 were used as target cells to evaluate cytotoxicity. 1 × 10⁶ cells/ml of H460, H3122, and K562 cell lines were cultured for 1 hour at 37°C with a culture medium added with 5 µl of calcein-AM (Invitrogen) and stained, and then centrifuged at 1000 rpm for 5 minutes and washed, respectively.

### [2-3] Evaluation of cytotoxicity

The target cells stained with calcein-AM in Example [2-2] were dispensed in a 96-well plate, respectively, and treated with the effector cells prepared in Example [2-1]. At this time, when NK92 cells were used as the effector cells, the NK92 cells were treated so that a ratio (E : T ratio) of effector cells to target cells was 20 : 1, and when primary NK cells were used as the effector cells, the primary NK cells were treated so that the E : T ratio was 10 : 1 and cultured at 37°C for 4 hours. The culture medium cultured above was centrifuged at 100 rcf for 5 minutes to separate the supernatant, and 100 µl each was dispensed into a 96-well black plate, and then ELISA assay (Fluorescence 480 nm/530 nm, SpectraMax i3x Molecular Device) was performed.

As a result, as illustrated in FIG. 1, it was confirmed that the cytotoxicity of NK92 cells was reduced by TGF-β for all three types of target cells, but the cytotoxicity of NK92 cells was recovered by the peptide of Example [1-5] or SB431542. In addition, the effect of recovering the cytotoxicity of the NK cells by the peptide of Examples [1-5] was equally confirmed not only in NK92 cells but also in primary NK cells as illustrated in FIG. 2.

### [Example 3]

### Recovery of sensitivity of NK cells to cancer cells decreased by TGF

How the peptide prepared in Examples [1-5] affected the sensitivity of NK cells to cancer cells decreased by TGF-β was confirmed.

To this end, first, 1 × 10⁵ cells/ml of a H460 lung cancer cell line was added with 20 µM the peptide of Example [1-5] or 5 µM a TGF-β signal inhibitor SB431542, respectively, cultured at 37°C for 2 hours, and then added with 10 ng/ml of TGF-β, and cultured again for 24 hours under the same conditions, respectively. Then, the cells were added with 20 µM the peptide of Example [1-5] or 5 µM SB431542, cultured at 37°C for 2 hours, and then added with 10 ng/ml of TGF-β, and cultured under the same conditions for 24 hours, respectively. 1 × 10⁶ cells/ml of the H460 cells cultured above were cultured for 1 hour at 37°C with a culture medium added with 5 µl of calcein-AM (Invitrogen) and stained, and then centrifuged at 1000 rpm for 5 minutes and washed to be prepared as 'target cells.'

As described above, the target cells stained with calcein-AM were dispensed in a 96-well plate, respectively, and treated with untreated (fresh) NK92 cells as effector cells at an E : T ratio of 20 : 1, and cultured at 37°C for 4 hours. The culture medium cultured above was centrifuged at 100 rcf for 5 minutes to separate the supernatant, and 100 µl each was dispensed into a 96-well black plate, and then ELISA assay (Fluorescence 480 nm/530 nm, SpectraMax i3x Molecular Device) was performed.

As a result, as illustrated in FIG. 3, it was confirmed that for the H460 cells treated with TGF-β, the NK cells did not show sufficient cytotoxicity, but for the H460 cells treated with the peptide of Example [1-5] or SB431542, the cytotoxicity of the NK cells was recovered again.

### [Example 4]

### Fratricide inhibition of NK cells

It was confirmed how TGF-β affected the fratricide between NK cells and how the peptide prepared in Example [1-5] affected the fratricide between the NK cells.

To this end, 1 × 10⁶ cells/ml of the NK92 cells cultured in the same manner as Example [2-1] were cultured for 1 hour at 37°C with a culture medium added with 5 µl of calcein-AM (Invitrogen) and stained, and then centrifuged at 1000 rpm for 5 minutes and washed to be prepared as 'target cells.' As described above, the target cells stained with calcein-AM were dispensed in a 96-well plate, respectively, and treated with untreated (fresh) NK92 cells or primary NK cells as effector cells at the ratio of 20 : 1 or 10 : 1 in the same manner as Example [2-3], and cultured at 37°C for 4 hours. The culture medium cultured above was centrifuged at 100 rcf for 5 minutes to separate the supernatant, and 100 µl each was dispensed into a 96-well black plate, and then ELISA assay (Fluorescence 480 nm/530 nm, SpectraMax i3x Molecular Device) was performed.

As a result, as illustrated in FIG. 4, it was confirmed that the fratricide activity of the NK cells was very high for the NK92 cells treated with TGF-β, but it was confirmed that the fratricide activity of the NK cells was significantly reduced by the peptide of Example [1-5] or SB431542.

### [Example 5]

### Inhibition of TGF-β expression in cancer cells

How the peptide prepared in Example [1-5] affected the expression of TGF-β in cancer cells was confirmed.

To this end, 2 × 10⁵ cells/ml of a lung cancer cell line H460 was cultured for 2 hours and stabilized, treated with the peptide of Example [1-5] at concentrations of 5 µM, 10 µM and 20 µM or treated with SB431542 at a concentration of 5 µM, cultured at 37°C for 24 hours, and then treated with the peptide of Example [1-5] or SB431542 again at the same concentrations, and then cultured at 37°C for 24 hours. The culture medium cultured above was centrifuged at 100 rcf for 5 minutes to separate the supernatant and the pellet, and then 100 µl each of the supernatant was dispensed into a 96-well black plate and ELISA assay was performed (Absorbance 450 nm, SpectraMax i3x Molecular Device), and the secretion amount of TGF-β was measured.

As a result, as illustrated in FIG. 5, it was confirmed that the amount of TGF-β secreted from the H460 cells was reduced depending on the concentration of the treated peptide of Example [1-5].

### [Example 6]

### Anticancer effect in animal model

To confirm the anticancer effect of the peptide prepared in Example [1-5] *in vivo,* 25 mg/kg of the peptide in Example [1-5] was intraperitoneally administered to a 24-month-old C57BL6J mouse 4 times once a week for 4 weeks. Then, 4 × 10⁵ B16F10 melanomas per mouse were administered through the tail vein, and the anticancer effect was confirmed by counting the number of nodules of cancer that had metastasized to the lung.

As a result, as illustrated in FIG. 6, it was confirmed that the number of nodules in the lungs of mice administered the peptide of Example [1-5] was significantly reduced compared to a negative control group.

Hereinabove, preferred embodiments of the present invention have been exemplarily described, but the scope of the present invention is not limited to the specific embodiments as described above, and can be changed appropriately by those skilled in the art within the scope described in the appended claims of the present invention.

## Claims

1. A composition for enhancing activity of NK cells comprising a peptide having an amino acid sequence of SEQ ID NO: 1 or a polynucleotide encoding the peptide as an active ingredient.

2. The composition for enhancing the activity of NK cells of claim 1, wherein the peptide inhibits expression or activity of TGF-β.

3. The composition for enhancing the activity of NK cells of claim 2, wherein the peptide enhances cytotoxicity of the NK cells.

4. The composition for enhancing the activity of NK cells of claim 2, wherein the peptide decreases fratricide activity of the NK cells.

5. The composition for enhancing the activity of NK cells of claim 1, wherein the composition is an anticancer drug or anticancer adjuvant.

6. The composition for enhancing the activity of NK cells of claim 5, wherein the cancer is solid cancer or metastatic cancer.

7. A pharmaceutical composition for preventing or treating cancer, comprising a peptide consisting of an amino acid sequence of SEQ ID NO: 1 or a polynucleotide encoding the peptide as an active ingredient.

8. The pharmaceutical composition for preventing or treating cancer of claim 7, wherein the peptide inhibits expression or activity of TGF-β in the cancer.

9. The pharmaceutical composition for preventing or treating cancer of claim 7, wherein the peptide improves susceptibility of the NK cells to cancer cells of the cancer.

10. The pharmaceutical composition for preventing or treating cancer of claim 7, wherein the cancer is overexpressed or overactivated TGF-β.

11. A cellular therapeutic agent for preventing or treating cancer comprising NK cells treated with a peptide having an amino acid sequence of SEQ ID NO: 1 or a polynucleotide encoding the peptide.

12. A method for enhancing activity of NK cells comprising treating NK cells with a peptide having an amino acid sequence of SEQ ID NO: 1 or a polynucleotide encoding the peptide.

13. A method for treating cancer comprising administering a pharmaceutical composition for preventing or treating cancer including a peptide consisting of an amino acid sequence of SEQ ID NO: 1 or a polynucleotide encoding the peptide as an active ingredient, to a subject.

14. A method for treating cancer comprising following steps:
(a) treating NK cells with a peptide consisting of an amino acid sequence of SEQ ID NO: 1 or a polynucleotide encoding the peptide; and
(b) administering the NK cells of step (a) to a subject.
